(19) Europäisches Patentamt / European Patent Office / Office européen des brevets

(11) EP 2 583 096 B1

(12) **EUROPÄISCHE PATENTSCHRIFT**

(45) Veröffentlichungstag und Bekanntmachung des Hinweises auf die Patenterteilung:
**10.04.2019 Patentblatt 2019/15**

(21) Anmeldenummer: **11725123.1**

(22) Anmeldetag: **16.06.2011**

(51) Int Cl.:
***G01N 33/22*** *(2006.01)*

(86) Internationale Anmeldenummer:
**PCT/EP2011/060052**

(87) Internationale Veröffentlichungsnummer:
**WO 2011/157801 (22.12.2011 Gazette 2011/51)**

(54) **VERFAHREN ZUM NACHWEIS VON PEROXIDISCHEN SPRENGSTOFFEN**

METHOD FOR DETECTING PEROXIDE EXPLOSIVES

PROCÉDÉ DE DÉTECTION D'EXPLOSIFS À BASE DE PEROXYDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.09.2010 DE 102010044756**
**18.06.2010 PCT/EP2010/058660**

(43) Veröffentlichungstag der Anmeldung:
**24.04.2013 Patentblatt 2013/17**

(73) Patentinhaber: **Rheinische Friedrich-Wilhelms-Universität Bonn**
**53113 Bonn (DE)**

(72) Erfinder:
- **WALDVOGEL, Siegfried**
**55218 Ingelheim (DE)**
- **SIERING, Carsten**
**53117 Bonn (DE)**
- **LUBCZYK, Daniel**
**53332 Bornheim (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**WO-A2-2004/035018 DE-A1-102009 029 787**
**US-A1- 2008 248 578 US-A1- 2008 248 578**
**US-A1- 2008 251 169**

- **PANDEY S: "Analytical applications of room-temperature ionic liquids: A review of recent efforts", ANALYTICA CHIMICA ACTA, ELSEVIER, AMSTERDAM, NL, Bd. 556, Nr. 1, 18. Januar 2006 (2006-01-18), Seiten 38-45, XP002613531, ISSN: 0003-2670, DOI: 10.1016/J.ACA.2005.06.038 [gefunden am 2005-07-20]**
- **MIYAKE A ET AL: "Mixing hazard evaluation of organic peroxides with other chemicals", JOURNAL OF LOSS PREVENTION IN THE PROCESS INDUSTRIES, ELSEVIER, UNITED KINGDOM, Bd. 18, Nr. 4-6, 1. Juli 2005 (2005-07-01) , Seiten 380-383, XP002613532, ISSN: 0950-4230, DOI: 10.1016/J.JLP.2005.06.021 [gefunden am 2005-08-01]**
- **SINGH R P ET AL: "Energetic nitrogen-rich salts and ionic liquids", ANGEWANDTE CHEMIE. INTERNATIONAL EDITION, WILEY VCH VERLAG, WEINHEIM, Bd. 45, Nr. 22, 26. Mai 2006 (2006-05-26), Seiten 3584-3601, XP002613533, ISSN: 1433-7851, DOI: 10.1002/ANIE.200504236 [gefunden am 2006-05-18]**
- **PANDEY S: "Analytical applications of room-temperature ionic liquids: A review of recent efforts", ANALYTICA CHIMICA ACTA, ELSEVIER, AMSTERDAM, NL, vol. 556, no. 1, 18 January 2006 (2006-01-18), pages 38-45, XP002613531, ISSN: 0003-2670, DOI: 10.1016/J.ACA.2005.06.038 [retrieved on 2005-07-20]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 583 096 B1

## Beschreibung

Die Erfindung betrifft ein Verfahren zum Nachweis von peroxidischen Sprengstoffen, sowie ein Kit umfassend die für dieses Verfahren benötigten Reagenzien.

## Hintergrund der Erfindung

Peroxidische Sprengstoffe wie das TATP oder HMTD zählen zu den so genannten Selbstelaboraten, die leicht in großer Menge aus Haushaltschemikalien hergestellt werden können. Die hohe Brisanz und damit schlechte Handhabbarkeit selbst von kleinen Mengen dieses Stoffes ist für die Probenbereitstellung des Echtstoffes eine sicherheitstechnische Herausforderung. In lipophilen ionischen Flüssigkeiten gelöst können die peroxidischen Sprengstoffe wie das TATP sicher gehandhabt werden.

Die hohe Brisanz von peroxidischen Sprengstoffen gestaltet die Analytik von aufgefundenen Substanzen äußerst gefährlich, da für eine zweifelsfreie Identifikation dieser pulverförmigen Feststoffe Material mechanisch entnommen werden muss. Getrocknete peroxidische Sprengstoffe wie TATP und im Besonderen HMTD können dabei gezündet werden. Üblicherweise werden derartige Substanzfunde mit Dieseltreibstoff überschüttet, nach einer gewissen Zeit kann der Sprengstoff gefahrlos mechanisch aufgesammelt werden. Aufgrund der Komplexität des aus mehreren tausend flüchtigen Komponenten bestehenden Dieselgemisches sind keine späteren Analysen mehr möglich und eine forensische Verwertung ist damit unmöglich.

Die Phlegmatisierung von Sprengstoffen zur besseren Verarbeitbarkeit - vor allem Nitroverbindungen - ist aus der US 20080251169 A1 bekannt. Weiterhin ist in S. Baj et al., Green Chemistry 8:292-295 (2006) die Verwendung von ionischen Flüssigkeiten in der Synthese von Dialkylperoxiden und in der WO2010/146170 die Verwendung von neutralen ionischen Flüssigkeiten zum Stabilisieren von peroxidischen Sprengstoffen wie TATP oder TATP-Hydrat, zum Herstellen stabiler Lösungen von peroxidischen Sprengstoffen und zur Phlegmatisierung dieser Sprengstoffe beschrieben. Ionische Flüssigkeiten sind dabei innovative Lösungsmittel mit einem vernachlässigbaren Dampfdruck (Ionic Liquids in Synthesis, Eds: P. Wasserscheidt, T. Welton, WILEY-VCH, Weinheim (2003)), die den den olfaktorischen Eindruck von TATP und anderen

peroxidischen Sprengstoffen nicht beeinflussen.

Aus US2008/0248578 ist ein Verfahren zur Analyse einer sprengstoffverdächtigen Probe bekannt, wobei Dampf oder Gas von der Probe in einem Gemisch aus verschiedenen Chemikalien aufgenommen wird und danach einer optische Analyse durchgeführt wird.

Für die forensische Analytik müssen insbesondere Proben von hochexplosiven peroxidischen Stoffen wie TATP oder HMTD sicher aufgenommen, gelagert und analysiert werden können, ohne die Probe selbst zu beeinflussen. Hierzu wird zurzeit, wie vorstehend angegeben Dieselöl verwendet, welches nicht nur geruchsintensiv ist und aufgrund der vielfältigen Inhaltsstoffe die Analyse stört, sondern auch keine Sicherheit bei der Handhabung bei peroxidischen Sprengstoffproben bietet.

## Kurzbeschreibung der Erfindung

Es wurde nun gefunden, dass durch Aufnahme von peroxidischen Sprengstoffen in speziellen Lösungen von ionischen Flüssigkeiten, wie sie aus der WO 04/035018 bekannt sind, sich eine stabile und leicht handhabbare Form der Explosivstoffe ergibt, die eine dramatisch reduzierte mechanische und thermische Empfindlichkeit aufweist, eine einfache Handhabung in konventionellen Laboratorien mit der üblichen Ausrüstung und somit eine Analyse und quantitative Bestimmung des Explosivstoffes erlaubt.

Die vorliegende Erfindung stellt ein sicheres Verfahren zum Nachweis peroxidischer Sprengstoffe zur Verfügung. Zur Probennahme von vermuteten Sprengstoffen werden dabei ausgewählte ionische Flüssigkeiten verwendet, da diese einen ausgezeichneten Schutz der Sprengstoffkristalle gegen Stoß und Elektrostatik bieten. Die Erfindung betrifft somit

(1) ein Verfahren zum Nachweis von peroxidischen Sprengstoffen umfassend Phlegmatisierung einer pulverförmigen Probe, die peroxidische Sprengstoffe enthalten kann, durch ihre Aufnahme in einem Gemisch aus wenigstens einer ionischen Flüssigkeit (nachfolgend kurz "IF"), die ausgewählt ist aus Tetrafluorborat-, Triflitimid-, Perfluoralkylsulfat-, Alkylsulfonat-, Arylsulfonat-, Perfluoralkylsulfonat-, bis-Perfluoralkylsulfonimid-, Acetat-, Alkylcarboxylat-, Isocyanat-, Isothiocyanat-, Thiosulfat-, Halogenid-, Borat-, Phosphat-, Nitrat- und Perchloratsalzen von *N*-alkylsubstituierten Stickstoffheterozyklen und wenigstens einem flüchtigen organischem Lösungsmittel, und analytischer Nachweis des in der erhaltenen IF-Probelösung phlegmatisierten peroxidischen Sprengstoffes;

(2) einen Kit für ein Nachweisverfahren wie in (1) definiert, umfassend eine Phlegmatisierungslösung bestehend aus wenigstens einer neuralen ionischen Flüssigkeit (IF) und wenigstens einem flüchtigen organischen Lösungsmittel wie in (1) definiert und eine oder mehrere Kalibrierungsproben enthaltend IF und darin phlegmatisierten, peroxidischen Sprengstoff; und

(3) die Verwendung eines Kits wie in (2) definiert für ein Nachweisverfahren wie in (1) definiert.

Die Erfindung stellt ein forensisches Arbeitsverfahren zur Verfügung, welches im Wesentlichen dadurch gekennzeichnet ist, dass verdächtige Stoffe im ersten Schritt in einem Gemisch aus IF und flüchtigem organischen Lösungsmittel ("IF-Lösung") aufgenommen oder vom Probenort mit der IF-Lösung abgewischt werden. Im zweiten Schritt wird die so erhaltene IF-Probenlösung der Analyse, z:b. GC/MS zugeführt, um die Sprengstoffart und optional auch dessen Gehalt zu bestimmen. Das erfindungsgemäße Verfahren zeichnet sich dadurch aus, dass der Sprengstoff in der erhaltenen IF-Probelösung phlegmatisiert ist, diese IF-Lösung die Analyse nicht stört und als Asservat sicher transportiert und gelagert werden kann.

**Kurzbeschreibung der Figuren**

Fig. 1: GC-Messung von TATP in einer Hexanlösung. Gerät: GC2010 der Fa. *Shimadzu,* Japan; Säule: Quarzkapillarsäule HP-5 der Fa. *Agilent,* USA (Länge: 30 m; Innendurchmesser: 0.25 μm); Trägergas: Wasserstoff; Injektortemperatur: 110 °C; Detektortemperatur: 310 °C; Säulenvordruck: 68.1 kPa; Programm: 50 °C Starttemperatur (3 min), 8 °C/min Heizrate, 100 °C Endtemperatur (6 min).

Fig. 2 : Mikroskopische Aufnahmen des Kristallbreis aus TATP in 1-Octyl-3-Methyl-imidazolium-tetrafluoroborat; A: 25fach, B: 100fach, C und D: 260fach vergrößert.

**Detaillierte Beschreibung der Erfindung**

In dem Verfahren gemäß Aspekt (1) der Erfindung und in dem Kit gemäß Aspekt (2) der Erfindung sind die lipophile ionische Flüssigkeiten von besonderer Bedeutung, da diese wenig Wasser aus der Umgebung aufnehmen. Da die Eigenschaften der ionischen Flüssigkeiten sowohl durch die Kationen als auch die Anionen bestimmt wird, können beide Seiten variiert werden. Vorzugsweise werden neutrale ionische Flüssigkeiten mit einer geringen Viskosität eingesetzt. Als lipophile Anionen eignen sich unter anderem Tetrafluoroborate, Triflitimide, Perfluoralkylsulfate, Alkylsulfonate, Arylsulfonate, Perfluoralkylsulfonate, bis-Perfluoralkylsulfonimide, Acetate, Alkylcarboxylate, Isocyanate, Isothiocyanate, Thiosulfate, Halogenide (einschließlich Iodide, Bromide, Chloride und Fluoride), Borate, Phosphate, Nitrate und Perchlorate, wobei Tetrafluorborate und Triflitimide besonders geeignet sind. Geeignete Kationen sind *N*-alkylsubstituierten Stickstoffheterozyklen, wie *N*-Alkylpyridinium-, *N*-Alkylpyrazinium-, *N*-Alkylpyridazinium-, *N*-Alkylpyrimidinium- und bis-*N*-Alkylimidazoliumionen, quatären Ammonium- und Phosphoniumionen, wobei *N,N*-Dialkylimidazolium- und *N*-Alkylpyridiniumionen besonders bevorzugt sind. Besonders bevorzugt sind dabei 1-Ethyl-3-methyl-imidazolium-bis(trifluormethansulfonimid), 1-Butyl-3-methylimidazolium-bis(tri-fluormethansulfonimid), 1-Hexyl-3-methylimidazolium-bis(trifluormethansulfonimid), 1-Ethyl-3-methylimidazolium-tetrafluoroborat, 1-Hexyl-3-methylimidazoliumtetrafluoroborat, 1-Octyl-3-methylimidazolium-tetrafluoroborat, 1-Decyl-3-methyl-imidazoilium-tetrafluoroborat, 1-Decyl-3-methylimidazoilium-tetrafluoroborat, *N*-Hexylpyridinium-tetrafluoroborat, *N*-Hexylpyridinium-bis(trifluomethansulfonimid), *N*-Butyl-3-methylpyridinium-tetrafluoroborat, *N*-Butyl-4-methylpyridinium-tetrafluoroborat und Gemische derselben.

Aufgrund der unpolaren Natur des TATP-Moleküls sind lipophile ionische Flüssigkeiten begünstigt. Diesen Trend kann man an der Löslichkeit von TATP in den 1-Alkyl-3-methylimidazoliumtetrafluoroboraten gut ablesen. Es ist daher bevorzugt, dass wenigstens ein Alkylrest des *N*,*N*-Dialkylimidazoliumions bzw. der *N*-Alkylrest des *N*-Alkylpyridiniums ein $C_6$ bis $C_{16}$-Alkylrest ist. Es können auch Gemische der genannten ionischen Flüssigkeit eingesetzt werden.

Peroxidische Sprengstoffe die gemäß der vorliegenden Erfindung bestimmt werden können sind dabei zyklische Peroxide wie Triacetontriperoxid (TATP), Hexamethylentriperoxiddiamin (HMTD), Diacetonperoxid usw. mit den Strukturen (I) bis (III)

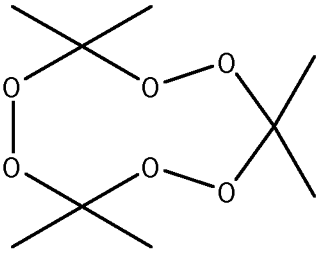

(I)

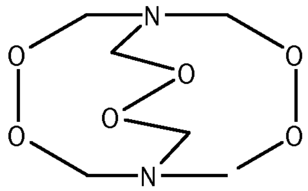

(II)

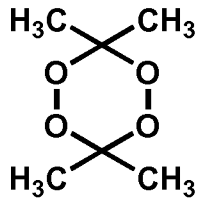

(III)

Diacylperoxide der nachfolgenden Formel (IV), worin R ein geradkettiger, verzweigter oder zyklischer, gesättigter $C_{1-5}$-Alkylrest oder ein mono oder polyzyklischer Arylrest ist, wobei die Alkyl- und Arylreste optional mit einem oder mehreren Resten ausgewählt aus Halogen, Nitro, Hydroxy und Oxo substituiert sein können, und wobei Diacetylperoxid und Bisbenzoylperoxid mit den Strukturen (Iva) und (IVb) besonders bevorzugt sind

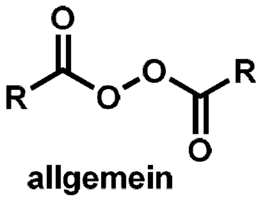

**allgemein**

(IV)

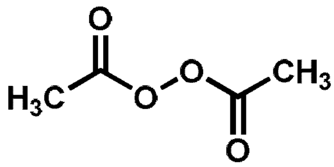

(IVa)

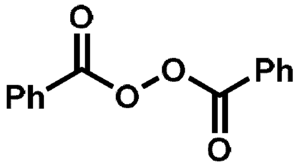

(IVb)

und sonstige leicht herstellbare Peroxide wie Bis(1-hydroxycyclohexyl)peroxidmit der nachfolgenden Formel (V)

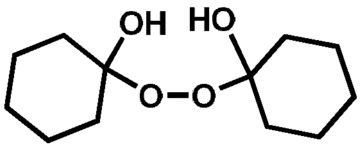

(V).

In dem Verfahren gemäß Aspekt (1) der Erfindung wird die IF als Gemisch mit wenigstens einem flüchtigen organischen Lösungsmittel eingesetzt. Geeignete flüchtige Lösungsmittel sind dabei schwer entzündliche, eine nachfolgende Analytik erlaubende Lösungsmittel einschließlich halogenierten Kohlenwasserstoffen wie z.B. Dichlormethan, Chloroform, Tetrachlorkohlenstoff, 1-Chlorbutan und 1,2-Dichlorethan, Toluol, Dimethylsulfoxid, Dimethylformamid, N-Methylpyrrolidon, Acetonitril, Essigester, Nitromethan, tert-Butanol, tert-Butylmethylether und Gemischen derselben, wobei Dichlormethan besonders bevorzugt ist. Der Gehalt der IF in dem flüchtigen Lösungsmittel beträgt dabei 1 bis 25 Gew.-%, vorzugsweise 5 bis 15 Gew.-%.

In einer besonderen Ausführungsform des Verfahrens von Aspekt (1) der Erfindung bildet nach der Probeaufnahme, durch Verdampfen des flüchtigen organischen Lösungsmittels, das IF mit dem peroxidischem Sprengstoff eine homogene kristalline Masse.

Das erfindungsgemäße Verfahren ist dabei sowohl zur qualitativen als auch zur quantitativen Analyse des peroxidischen Sprengstoffes geeignet. Zur quantitative Analyse kann zusätzlich ein Abgleich mit einer Kalibrierungsprobe enthaltend IF und peroxidischen Sprengstoff erfolgen.

Der Kit von Aspekt (2) der Erfindung kann weiterhin eine oder mehrere Kalibrierungsproben, die eine IF und peroxidischem Sprengstoff enthalten, aufweisen. Diese Kalibrierungsproben können dabei eine homogene kristalline Masse wie vorstehend definiert sein.

Die Erfindung wird anhand der folgenden Beispiele näher erläutert, die jedoch den Schutzbereich der Erfindung nicht einschränken.

## Beispiele

Beispiel 1: Herstellung einer mit Peroxid/TATP gesättigten ionischen Flüssigkeit. 20 bis 50 ml der ionischen

Flüssigkeit wird in einen 250 ml Kolben mit einem Magnetrührerkern gegeben. Vorzugsweise hat der Kolben eine weite Öffnung, um pulverförmiges Material leicht zugeben zu können, und der Boden sollte konisch geformt sein, um eine Dekantierung der Flüssigkeit nach der Auflösung zu erleichtern. Es muss beachtet werden, dass der Rührkern und der Magnetrührer genügend starken Magnetfelder besitzen, um auch zähflüssige Suspensionen zuverlässig mit hoher Drehzahl mischen zu können. Der Kolben wird auf einen Magnetrührer mit Stativ nahe an den Magneten gespannt. Der Rührer wird auf eine möglichst hohe Drehzahl eingestellt, so dass der Rührkern der Drehung noch zuverlässig folgen kann. In der Praxis beträgt die Drehzahl zwischen 700 und 1000 U/min. 500 mg des Peroxides/Triacetonperoxid-Hydrat (TATP) wird zugegeben, und der Kolben wird gasdicht verschlossen. Der Schliffstopfen wird mit Hochvakuumfett sorgfältig abgedichtet und zugeklemmt, damit kein Peroxid während des Auflösens entweichen kann. Das Gemisch wird so langegerührt, bis das Peroxid vollständig aufgelöst ist. Dies dauert i.A. 8 bis 12 h. Falls das Peroxid nicht vollständig aufgelöst wurde, wird noch mindestens 48 h weiter gemischt, um sicherzustellen, dass die Lösung gesättigt ist. Falls das Peroxid vollständig gelöst wurde, wird ein zusätzlicher 500 mg Ansatz TATP zugegeben und wieder entweder bis vollständiger Auflösung oder 12 h gemischt. Dieser Vorgang wird so oft wiederholt, bis ungelöstes Peroxid in der Lösung verbleibt. Danach wird noch 48 h weiter gerührt.

Wenn die Lösungen gesättigt sind, wird der Rührer ausgeschaltet, und die Lösungen werden für mindestens 24 h stehen gelassen. Dabei sedimentiert das überschüssige Peroxid entweder auf der Oberfläche oder auf dem Boden - je nach Dichte der Flüssigkeit. Die klare Flüssigkeit wird mit einer Pipette sehr langsam abgesaugt und ins dichte Gebinde abgefüllt. Die Peroxidrückstände im Auflösungskolben werden mit 5 % Natriumdithionit in einem Aceton-Wassergemisch (70:30, Gewichtsanteile) entsorgt.

Es ist zu beachten, dass eine Filtrierung mit den zähflüssigen ionischen Flüssigkeiten ohne große Materialverluste nicht möglich ist. Daher muss das überschüssige Peroxid durch Dekantieren oder Zentrifugieren getrennt werden. Es bleiben dabei noch geringe Mengen von sehr kleinen Kristallen in der Flüssigkeit zurück. Dieser sehr kleine Überschuss stellt sicher, dass die TATP-Lösungen gesättigt bleiben, auch wenn kleine Mengen Acetonperoxid z.B. durch die Gebindedichtungen entweichen. Die Kristallreste stellen auch keine Gefahr durch erhöhte Empfindlichkeit dar, weil sie nur in so kleinen Mengen vorhanden sind. Die Eigenschaften der TATP-haltigen ionischen Flüssigkeiten sind in der nachfolgenden Tabelle 1 zusammengefasst.

Tab. 1: Übersicht über die Löslichkeit von TATP in einigen Ionischen Flüssigkeiten (IF)

| Ionische Flüssigkeit (IF) | M (IF) [g/mol] | rel.TATP-Integral | c[TATP] (w%) |
|---|---|---|---|
| 1-Ethyl-3-methylimidazolium-bis(trifluormethansulfonimid) | 391,31 | 0,12 | 0,38 |
| 1-Butyl-3-methylimidazolium-bis(trifluormethansulfonimid) | 419,36 | 0,32 | 0,94 |
| 1-Hexyl-3-methylimidazolium-bis(trifluormethansulfonimid) | 447,42 | 0,31 | 0,86 |
| 1-Ethyl-3-methylimidazolium-tetrafluoroborat | 197,97 | 0,02 | 0,12 |
| 1-Hexyl-3-methylimidazolium-tetrafluoroborat | 254,08 | 0,27 | 1,31 |
| 1-Octyl-3-methylimidazolium-tetrafluoroborat | 282,13 | 0,49 | 2,14 |
| 1-Hexyl-3-methylimidazolium-iodid | 294,18 | 0,09 | 0,38 |
| 1-Decyl-3-methylimidazoiliumtetrafluoroborat | 310,18 | 0,32 | 1,27 |
| N-Hexylpyridinium-tetrafluoroborat | 251,07 | 0,09 | 0,44 |
| N-Hexylpyridinium-bis(trifluomethansulfonimid) | 444,41 | 0,33 | 0,92 |
| N-Butyl-3-methylpyridinium-tetrafluoroborat | 237,05 | 0,06 | 0,31 |
| N-Butyl-4-methylpyridinium-tetrafluoroborat | 237,05 | 0,00 | 0,00 |

Beispiel 2: Bestimmung des TATP- bzw. HMTD-Gehaltes *via* NMR-Sepktroskopie.

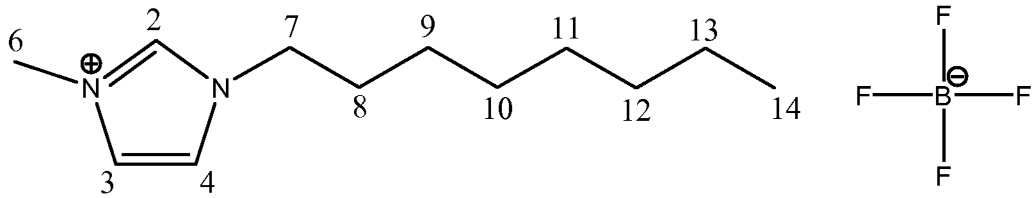

*1*-Octyl-*3*-methylimidazoliumtetrafluoroborat
$^1$H-NMR (400 MHz, DMSO-*d6*): δ = 0.85 (t, 3H, H 14, $^3$J = 8.0 Hz), 1.25 (m, 10H, H 9-13), 1.36 (s, 0.49H, $CH_3$ TATP), 1.79 (m, 2H, H 8), 3.85 (s, 3H, H 6), 4.15 (t, 2H, H 7, $^3$J = 8.0 H), 7.70 (m, 2H, H3-4), 9.04(s, 1H, H 2).

Zur Konzentrationsbestimmung wird das TATP-Signal bei 1.36 ppm mit dem Signal der Methylgruppe des Imidazols bei 3.85 ppm in Relation gesetzt.
c (Gew% TATP) = [M (TATP) • (TATP Signal/6) • 100] / M (IF) (1),
wobei M (TATP) = 222,2 g/mol beträgt.

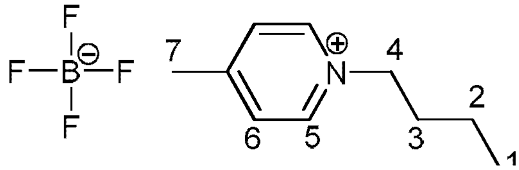

*N*-Butyl-4-methylpyridinium-tetrafluoroborat: $^1$H-NMR (400 MHz, DMSO-*d6*): δ = 0.88 (t, 3H, H 1, $^3J_{1,2}$ = 8.0 Hz), 1.25 (tq, 2H, H 2, $^3J_{2,1}$ = 8.0 Hz, $^3J_{2,3}$ = 8.0 Hz), 1.87 (tt, 2H, H 3, $^3J_{3,2}$ = 8.0 Hz, $^3J_{3,4}$ = 6.0 Hz), 2.60 (s,3H, H 7), 4.52 (t, 2H, H 4, $^3J_{4,3}$ = 6.0 Hz), 4.65 (d, 0.35H, $H_A$ HMTD, $^3J_{HA,HB}$ = 14.0 Hz), 4.77 (d, 0.35H, $H_B$ HMTD, $^3J_{HB,HA}$ = 14.0 Hz), 7.95 (d, 2H, H 6, $^3J_{6,5}$ = 4.0 Hz), 8.86 (d, 2H, H 5, $^3J_{5,6}$ = 8.0 Hz).

Zur Konzentrationsbestimmung von HMTD wird die Summe der Integrale bei 4.65 ppm und 4.77 ppm (entspricht 12 Protonen) mit dem Signal der Methylgruppe der Alkylkette bei 0.88 ppm (entspricht 3 Protonen) in Relation gesetzt. Zur Vereinfachung werden schon während der Auswertung die Integrale so eingepasst, dass für das Integral der IF-Methylprotonen (0.88 ppm) ein Wert von 3 erhalten wird.

$$c(HMTD) = \frac{HMTD - Signal/12}{IL - Signal/3} * \frac{M(HMTD)}{M(IF)}$$

Wegen der Normierung auf die Methyl-Protonen der IF wird der linke untere Term 1. Für die Darstellung in % wird mit 100 multipliziert.

c (HMTD)=[M (HMDT) · (HMTD Signal/12) · 100] / M (IF) (1),

wobei M (HMTD) = 208,10 g/mol beträgt.

Tabelle 2: Übersicht über die Löslichkeit von HMTD in ionischen Flüssigkeiten (IF)

| Ionische Flüssigkeit (IF) | M (IF) [g/mol] | rel. HMTD-Integral[a] | c[HMTD] (w%) |
|---|---|---|---|
| 1-Ethyl-3-methylimidazolium-bis(trifluormethansulfon)imid | 391,31 | 0,54 | 2,39 |
| 1-Butyl-1-methylpyrolidinium-bis(trifluormethansulfon)imid | 422,41 | 0,55 | 2,26 |
| *N*-Hexylpyridinium-bis(trifluormethansulfon)imid | 444,41 | 0,54 | 2,11 |
| *N*-Butyl-3-methylpyridinium-tetrafluoroborat | 237,05 | 0,23 | 1,68 |
| *N*-Butyl-3-methylpyridinium-tetrafluoroborat | 237,05 | 0,17 | 1,24 |
| *N*-Butyl-4-methylpyridinium-tetrafluoroborat | 237,05 | 0,70 | 5,12 |
| *N*-Hexylpyridiniumtetrafluoroborat | 251,07 | 0,04 | 0,28 |

(fortgesetzt)

| Ionische Flüssigkeit (IF) | M (IF) [g/mol] | rel. HMTD-Integral[a] | c[HMTD] (w%) |
|---|---|---|---|
| 1-Hexyl-3-methylimidazolium-bis(trifluormethansulfon) imid | 447,42 | 1,17 | 4,53 |
| 1-Ethyl-3-methylimidazolium-diethylphosphat | 264,26 | 0,14 | 0,92 |
| 1-Ethyl-3-methylimidazolium-methansulfonat | 206,26 | 0,48 | 4,04 |
| 1-Butyl-1-methylpyrrolidiniumtrifluormethansulfonat | 291,34 | 0,17 | 1,01 |
| [a] Zur Berechnung des relativen Signals werden im NMR die Integrale durch Normierung einer Methylgruppe der IF auf 3 eingestellt. Das relative Signal ist die Summe von beiden HMTD-Protonensignalen nach dieser Normierung und repräsentiert somit das Integral der 12 HMTD-Protonen pro Molekül IF (s.o.). | | | |

Beispiel 3: Phlegmatisierung peroxidischer Sprengstoffe.

*Phlegmatisierungs-Lösung:* 10 % 1-Hexyl-3-methylimidazolium bis(trifluormethyl-sulfon)imid in Dichlormethan

Vor Phlegmatisierungsbehandlung:

Reibempfindlichkeit: HMTD 0,05 N - 100% Zündung; TATP 0,2 N - 100% Zündung.

Schlagempfindlichkeit: HMTD 0,2 J -100% Zündung, TATP 0,5 J - 100% Zündung. Phlegmatisierungsversuche: 100 mg Sprengstoff werden mit 200 mg 10% Phlegmatisierungslösung angefeuchtet, 15 min trocknen gelassen und dann vermessen. Im Rahmen des Messbereichs konnten die beiden phlegmatisierten

Sprengstoffe nicht mehr gezündet werden: >1 J Schlag- und >30 N Reibempfindlichkeit.

Beispiel 4: Gefahrlosen Probenahme von TATP

Zur Probenahme wird die Probe mit der Desensibilisierungslösung behandelt. Hierzu werden 200 mg trockenes TATP mit 5 ml einer Lösung aus 10% 1-Methyl-3-octylimidazoliumtetrafluoroborat in Dichlormethan betropft oder besprüht. Nach 5 min Einwirkung wird aus dem farblosen Kristallpulver eine durchscheinende opake Masse. Dieser "Kristallbrei" kann durch Schlag, Elektrostatik oder Reibung nicht mehr gezündet werden. Eine Probe kann gefahrlos mit einem Spatel genommen werden. Für eine analytische Auswertung/Identifikation kann diese via $^1$H-NMR-Spektroskopie oder gaschromatographisch ausgewertet werden. Zur GC-Analytik wird ein kleiner Teil der Probe (ca. 20 mg) mit etwas Hexan überschichtet und die unpolare Phase direkt in das GC-Ofen eingespritzt. Anhand der Retentionszeit konnte eindeutig TATP nachgewiesen/identifiziert werden. Das GC-Spektrum ist in Fig. 1 gezeigt.

Die genommene TAT-Probe ist über längere Zeit (> 2 Monate) stabil. Eine Aufbewahrung bei Raumtemperatur oder bis 40°C ist möglich und beeinflusst das analytische Ergebnis nicht. Werden die Proben unterhalb der Raumtemperatur aufbewahrt (4-10 °C) so kommt es nicht zu einer Ausbildung von großen Kristallen, die leicht zu zünden wären, sondern zu einem nahezu kolloidalen Niederschlag, der sich fast nicht durch Filtration abtrennen lässt. Mit der Zeit wandelt sich Großteils der ursprünglichen Probe in diese mikrokristalline TATP-Variante um.

Aus Fig. 2 ist ersichtlich, dass sich eine Masse aus ähnlich großen Kristallen mit einer Größe von 20 $\mu$m bildet. Die geringe Größe und nicht auftretende Reaggregation der Kristalle beruht wahrscheinlich auf der Wirkung der ionischen Flüssigkeit als Tensid. Hierbei lagern sich die Moleküle der der ionischen Flüssigkeit mittels ihrer unpolaren Seitenkette an die Kristalle an. Dies erklärt die hohe Dispersion in der ionischen Flüssigkeit. Für den hier untersuchten Kristallbrei wurde mittels NMR-Spektroskopie eine Konzentration von ca. 30 % TATP bestimmt. Der so erhaltene Kristallbrei kann selbst nach unsachgemäßer Handhabung (Exposition, Sonne Hitze, Kälte) noch eindeutig forensisch ausgewertet werden.

## Patentansprüche

1. Verfahren zum Nachweis von peroxidischen Sprengstoffen umfassend Phlegmatisierung einer pulverförmigen Probe, die peroxidische Sprengstoffe enthalten kann, durch ihre Aufnahme in einem Gemisch aus wenigstens einer ionischen Flüssigkeit (IF), die ausgewählt ist aus Tetrafluorborat-, Triflitimid-, Perfluoralkylsulfat-, Alkylsulfonat-, Arylsulfonat-, Perfluoralkylsulfonat-, bis-Perfluoralkylsulfonimid-, Acetat-, Alkylcarboxylat-, Isocyanat-, Isothiocyanat-, Thiosulfat-, Halogenid-, Borat-, Phosphat-, Nitrat- und Perchloratsalzen von *N*-alkylsubstituierten Stickstoffheterozyklen und wenigstens einem flüchtigen organischem Lösungsmittel, und
analytischer Nachweis des in der erhaltenen IF-Probelösung phlegmatisierten peroxidischen Sprengstoffes.

**2.** Verfahren nach Anspruch 1, wobei der Nachweis mittels spektroskopischer oder chromatographischer Verfahren, vorzugsweise mittels $^1$H-NMR-Spektroskopie oder Gaschromatographie erfolgt.

**3.** Verfahren nach Anspruch 1 oder 2, wobei die Salze von *N*-alkylsubstituierten Stickstoffheterozyklen ausgewählt sind aus *N*-Alkylpyridinium, *N*-Alkylpyrazinium, *N*-Alkylpyridazinium, *N*-Alkylpyrimidinium und bis-*N*-Alkylimidazolium, quatären Ammonium und Phosphoniumsalzen, wobei Tetrafluorborate und bis-Perfluoralkylsulfonimide von 1,3-bis-*N*-Alkylimidazolium- und *N*-Alkylpyridiniumsalzen besonders bevorzugt sind.

**4.** Verfahren nach Anspruch 3, wobei die wenigstens eine IF ausgewählt ist aus 1-Ethyl-3-methylimidazolium-bis(trifluormethansulfonimid), 1-Butyl-3-methylimidazolium-bis(trifluormethansulfonimid), 1-Hexyl-3-methylimidazolium-bis(tri-fluormethansulfonimid), 1-Ethyl-3-methylimidazolium-tetrafluoroborat, 1-Hexyl-3-methylimidazolium-tetrafluoroborat, 1-Octyl-3-methylimidazolium-tetrafluoroborat, 1-Decyl-3-methylimidazoilium-tetrafluoroborat, 1-Decyl-3-methylimidazolium-tetrafluoroborat, *N*-Hexylpyridinium-tetrafluoroborat, *N*-Hexylpyridinium-bis(trifluomethansulfonimid), *N*-Butyl-3-methylpyridinium-tetrafluoroborat und *N*-Butyl-4-methylpyridinium-tetrafluoroborat.

**5.** Verfahren nach einem oder mehreren der Ansprüche 1 bis 4, wobei das wenigstens eine flüchtige organische Lösungsmittel ein schwer entzündliches, die Analytik erlaubendes Lösungsmittel ist.

**6.** Verfahren nach Anspruch 5, wobei das wenigstens eine flüchtige organische Lösungsmittel ausgewählt ist aus halogenierten Kohlenwasserstoffen, wie z.B. Dichlormethan, Chloroform, Tetrachlorkohlenstoff, 1-Chlorbutan und 1,2-Dichlorethan, Toluol, Dimethylsulfoxid, Dimethylformamid, N-Methylpyrrolidon, Acetonitril, Essigester, Nitromethan, tert-Butanol, tert-Butylmethylether und Gemischen derselben, wobei Dichlormethan besonders bevorzugt ist.

**7.** Verfahren nach einem oder mehreren der Ansprüche 1 bis 6, wobei das Gemisch aus wenigstens einer IF und dem wenigstens einem flüchtigen organische Lösungsmittel eine homogene Lösung ist, wobei vorzugsweise der Gehalt an IF in dem Gemisch 1 bis 25 Gew.-%, vorzugsweise 5 bis 15 Gew.-% beträgt.

**8.** Verfahren nach Anspruch 7, wobei nach Probeaufnahme, durch Verdampfen des flüchtigen organischen Lösungsmittels, das IF mit dem peroxidischem Sprengstoff eine homogene kristalline Masse bildet.

**9.** Verfahren nach einem oder mehreren der Ansprüche 1 bis 8, wobei der peroxidische Sprengstoff ausgewählt ist aus Triacetontriperoxid (TATP), Hexamethylentriperoxiddiamin (HMTD), Diacetonperoxid, Diacylperoxiden der Formel R(C=O)-OO-(C=O)R (worin R ein geradkettiger, verzweigter oder zyklischer, gesättigter $C_{1-5}$-Alkylrest oder ein mono oder polyzyklischer Arylrest ist, wobei die Alkyl- und Arylreste optional mit einem oder mehreren Resten ausgewählt aus Halogen, Nitro, Hydroxy, Oxo substituiert sein können) und sonstige leicht herstellbare Peroxide wie Bis(1-hydroxycyclohexyl)peroxid, und wobei TATP und HMTD besonders bevorzugt sind.

**10.** Verfahren nach einem oder mehreren der Ansprüche 1 bis 9, das zur qualitativen und quantitativen Analyse des peroxidischen Sprengstoffes geeignet ist.

**11.** Verfahren nach Anspruch 10, wobei zur quantitative Analyse zusätzlich ein Abgleich mit einer Kalibrierungsprobe enthaltend IF und peroxidischen Sprengstoff erfolgt.

**12.** Kit für ein Nachweisverfahren gemäß einem oder mehreren der Ansprüche 1 bis 11 umfassend eine Phlegmatisierungslösung bestehend aus wenigstens einer neutralen ionischen Flüssigkeit (IF) und wenigstens einem flüchtigen organischen Lösungsmittel wie in Ansprüchen 1 bis 7 definiert und eine oder mehrere Kalibrierungsproben enthaltend IF und darin phlegmatisierten, peroxidischem Sprengstoff.

**13.** Kit nach Anspruch 12, wobei die eine oder mehrere Kalibrierungsproben eine homogene kristalline Masse wie in Anspruch 8 definiert umfasst.

14. Verwendung eines Kits nach Anspruch 12 oder 13 für ein Nachweisverfahren gemäß einem oder mehreren der Ansprüche 1 bis 11.

15. Verwendung nach Anspruch 14, wobei der Kit weiterhin eine oder mehrere Kalibrierungsproben enthaltend IF und peroxidischem Sprengstoff, vorzugsweise eine homogene kristalline Masse wie in Anspruch 8 definiert umfasst.

## Claims

1. A process for detecting peroxidic explosives, comprising
the phlegmatization of a powdery sample that may contain peroxidic explosives by including it in a mixture of at least one ionic liquid (IL) selected from tetrafluoroborate, triflitimide, perfluoroalkylsulfate, alkylsulfonate, arylsulfonate, perfluoroalkylsulfonate, bis(perfluoroalkyl)sulfonimide, acetate, alkylcarboxylate, isocyanate, isothiocyanate, thiosulfate, halide, borate, phosphate, nitrate, and perchlorate salts of N-alkylsubstituted nitrogen heterocycles and at least one volatile organic solvent; and
analytical detection of the peroxidic explosive phlegmatized in the IL sample solution obtained.

2. The process according to claim 1, wherein said detection is performed by using spectroscopic or chromatographic methods, preferably by means of $^1$H NMR spectroscopy or gas chromatography.

3. The process according to claim 1 or 2, wherein said salts of N-alkylsubstituted nitrogen heterocycles are selected from N-alkylpyridinium, N-alkylpyrazinium, N-alkylpyridazinium, N-alkylpyrimidinium and bis-N-alkylimidazolium, quaternary ammonium and phosphonium salts, wherein tetrafluoroborates and bis(perfluoroalkyl)sulfonimides of 1,3-bis(N-alkyl)-imidazolium and N-alkylpyridinium salts are particularly preferred.

4. The process according to claim 3, wherein said at least one IL is selected from 1-ethyl-3-methylimidazolium bis(trifluoromethane) sulfonimide, 1-butyl-3-methylimidazolium bis(trifluoromethane)sulfonimide, 1-hexyl-3-methylimidazolium bis(trifluoromethane) sulfonimide, 1-ethyl-3-methylimidazolium tetrafluoroborate, 1-hexyl-3-methylimidazolium tetrafluoroborate, 1-octyl-3-methylimidazolium tetrafluoroborate, 1-decyl-3-methylimidazolium tetrafluoroborate, 1-decyl-3-methylimidazolium tetrafluoroborate, N-hexylpyridinium tetrafluoroborate, N-hexylpyridinium bis(trifluoromethane)sulfonimide, N-butyl-3-methylpyridinium tetrafluoroborate, and N-butyl-4-methylpyridinium tetrafluoroborate.

5. The process according to one or more of claims 1 to 4, wherein said at least one volatile organic solvent is a solvent that is not readily flammable and allows for analysis.

6. The process according to claim 5, wherein said at least one volatile organic solvent is selected from halogenated hydrocarbons, such as dichloromethane, chloroform, carbon tetrachloride, 1-chlorobutane and 1,2-dichloroethane, toluene, dimethylsulfoxide, dimethylformamide, N-methylpyrrolidone, acetonitrile, ethyl acetate, nitromethane, tert-butanol, tert-butyl methyl ether, and mixtures thereof, wherein dichloromethane is particularly preferred.

7. The process according to one or more of claims 1 to 6, wherein said mixture of at least one IL and said at least one volatile organic solvent is a homogeneous solution, wherein preferably the content of IL in said mixture is from 1 to 25% by weight, preferably from 5 to 15% by weight.

8. The process according to claim 7, wherein, after the sample has been included, said IL forms a homogeneous crystalline mass with said peroxidic explosive upon evaporation of said volatile organic solvent.

9. The process according to one or more of claims 1 to 8, wherein said peroxidic explosive is selected from triacetone triperoxide (TATP), hexamethylene triperoxide diamine (HMTD), diacetone peroxide, diacyl peroxides of the formula R(C=O)-OO-(C=O)R (wherein R is a straight-chain, a branched or a cyclic, saturated $C_{1-5}$-alkyl radical or a monocyclic or a polycyclic aryl radical, wherein said alkyl and aryl radicals may optionally be substituted by one or more radicals selected from halogen, nitro, hydroxy, oxo, and other peroxides that can be easily produced, such as bis(1-hydroxycyclohexyl)peroxide, and wherein TATP and HMTD are particularly preferred.

10. The process according to one or more of claims 1 to 9 that is suitable for the qualitative and quantitative analysis of said peroxidic explosive.

11. The process according to claim 10, wherein a comparison with a calibration sample containing IL and peroxidic explosive is additionally performed for said quantitative analysis.

12. A kit for a detection process according to one or more of claims 1 to 11, comprising a phlegmatization solution consisting of at least one neutral ionic liquid (IL) and at least one volatile organic solvent as defined in claims 1 to 7, and one or more calibration samples containing IL and a peroxidic explosive phlegmatized therein.

13. The kit according to claim 12, wherein said one or more calibration samples comprise a homogeneous crystalline mass as defined in claim 8.

14. Use of a kit according to claim 12 or 13 for a detection process according to one or more of claims 1 to 11.

15. The use according to claim 14, wherein said kit further comprises one or more calibration samples containing IL and a peroxidic explosive, preferably a homogeneous crystalline mass as defined in claim 8.

**Revendications**

1. Procédé de détection d'explosifs à base de peroxyde comprenant la flegmatisation d'un échantillon pulvérulent susceptible de contenir des explosifs à base de peroxyde, par son absorption dans un mélange constitué d'au moins un liquide ionique (IF) qui est choisi parmi des sels tétrafluoroborate, triflitimide, perfluoroalkylsulfate, alkylsulfonate, arylsulfonate, perfluoroalkylsulfonate, bis-perfluoroalkylsulfonimide, acétate, alkylcarboxylate, isocyanate, isothiocyanate, thiosulfate, halogénure, borate, phosphate, nitrate et perchlorate d'hétérocycles d'azote substitués par un groupe *N*-alkyle et au moins un solvant organique volatil, et
la détection analytique de l'explosif à base de peroxyde flegmatisé dans la solution d'IF-échantillon obtenue.

2. Procédé selon la revendication 1, dans lequel la détection s'effectue au moyen d'un procédé spectroscopique ou chromatographique, de préférence au moyen d'une spectroscopie RMN-$^1$H ou d'une chromatographie en phase gazeuse.

3. Procédé selon la revendication 1 ou 2, dans lequel les sels d'hétérocycles d'azote substitués par un groupe *N*-alkyle sont sélectionnés parmi le *N*-alkylpyridinium, le *N*-alkylpyrazinium, le *N*-alkyl-pyridazinium, le *N*-alkylpyrimidinium et le bis-*N*-alkylimidazolium, l'ammonium quaternaire et des sels de phosphonium, dans lequel les tétrafluoroborates et les bis-perfluoroalkylsulfonimides de sels de 1,3-bis-*N*-alkylimidazolium et de *N*-alkylpyridinium sont particulièrement préférés.

4. Procédé selon la revendication 3, dans lequel l'IF, au moins au nombre de un, est choisi parmi le 1-éthyl-3-méthylimidazolium-bis(trifluorométhane-sulfonimide), le 1-butyl-3-méthylimidazolium-bis(trifluorométhane-sulfonimide, le 1-hexyl-3-méthylimidazolium-bis(trifluorométhane-sulfonimide), le 1-éthyl-3-méthylimidazolium-tétrafluoroborate, le 1-hexyl-3-méthylimidazolium-tétrafluoroborate, le 1-octyl-3-méthylimidazolium-tétrafluoroborate, le 1-décyl-3-méthylimidazolium-tétrafluoroborate, le 1-décyl-3-méthylimidazolium-tétrafluoroborate, le *N*-hexylpyridinium-tétrafluoroborate, le *N*-hexylpyridinium-bis(trifluorométhane-sulfonimide), le *N*-butyl-3-méthylpyridinium-tétrafluoroborate et le *N*-butyl-4-méthylpyridinium-tétrafluoroborate.

5. Procédé selon une ou plusieurs des revendications 1 à 4, dans lequel le solvant organique volatil, au moins au nombre de un, est un solvant difficilement inflammable permettant l'analyse.

6. Procédé selon la revendication 5, dans lequel le solvant organique volatil, au moins au nombre de un, est choisi parmi des hydrocarbures halogénés, comme par exemple le dichlorométhane, le chloroforme, le tétrachlorure de carbone, le 1-chlorobutane et le 1,2-dichloroéthane, le toluène, le diméthylsulfoxyde, le diméthylformamide, la N-méthylpyrrolidone, l'acétonitrile, l'ester acétique, le nitrométhane, le tert-butanol, le tert-butylméthyléther et des mélanges de ceux-ci, le dichlorométhane étant particulièrement préféré.

7. Procédé selon une ou plusieurs des revendications 1 à 6, dans lequel le mélange est une solution homogène constituée d'au moins un IF et d'au moins un solvant organique volatil, dans lequel de préférence la teneur en IF dans le mélange est de 1 à 25 % en poids, de préférence de 5 à 15 % en poids.

8. Procédé selon la revendication 7, dans lequel après le prélèvement d'échantillon, l'IF forme, par évaporation du

solvant organique volatil, une masse cristalline homogène avec l'explosif à base de peroxyde.

9. Procédé selon une ou plusieurs des revendications 1 à 8, dans lequel l'explosif à base de peroxyde est choisi parmi le triperoxyde de triacétone (TATP), le triperoxyde d'hexaméthylène-diamine (HMTD), le peroxyde de diacétone, les peroxydes de diacyle de formule R(C=O)-OO-(C=O)R (dans laquelle R est un radical alkyle en $C_1$ à $C_5$ saturé, à chaîne linéaire, ramifié ou cyclique, ou un radical aryle mono- ou polycyclique, où les radicaux alkyle et aryle peuvent éventuellement être substitués par un ou plusieurs radicaux choisis parmi les groupes halogéno, nitro, hydroxy, oxo) et d'autres peroxydes pouvant être produits facilement tels que le bis(1-hydroxycylohexyl)peroxyde, le TATP et le HMTD étant particulièrement préférés.

10. Procédé selon une ou plusieurs des revendications 1 à 9, qui est approprié pour l'analyse qualitative et quantitative de l'explosif à base de peroxyde.

11. Procédé selon la revendication 10, dans lequel pour l'analyse quantitative, on effectue de plus une comparaison avec un échantillon d'étalonnage contenant de l'IF et un explosif à base de peroxyde.

12. Kit de réalisation d'un procédé de détection selon une ou plusieurs des revendications 1 à 11, comprenant une solution de flegmatisation composée d'au moins un liquide ionique neutre (IF) et d'au moins un solvant organique volatil tel que défini dans les revendications 1 à 7, et un ou plusieurs échantillons d'étalonnage contenant de l'IF et un explosif à base de peroxyde flegmatisé dans celui-ci.

13. Kit selon la revendication 12, dans lequel le ou les échantillons d'étalonnage contiennent une masse cristalline homogène telle que définie dans la revendication 8.

14. Utilisation d'un kit selon la revendication 12 ou 13, pour réaliser un procédé de détection selon une ou plusieurs des revendications 1 à 11.

15. Utilisation selon la revendication 14, dans laquelle le kit comprend en outre un ou plusieurs échantillons d'étalonnage contenant de l'IF et un explosif à base de peroxyde, de préférence une masse cristalline homogène telle que définie dans la revendication 8.

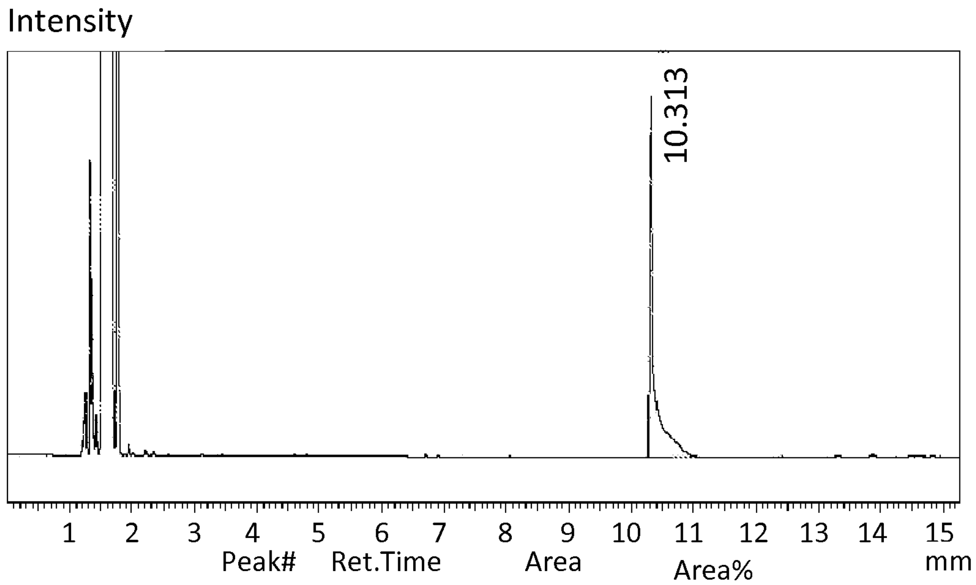


| Peak# | Ret.Time | Area | Area% |
|---|---|---|---|
| 1 | 10.313 | 410437 | 100.0000 |
| Total | | 410437 | 100.0000 |

**Fig.1**

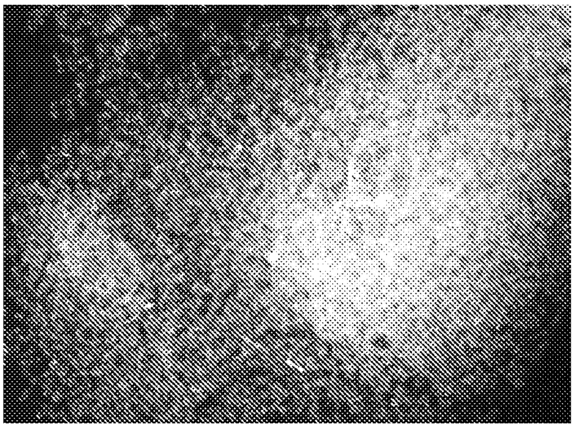

**A**

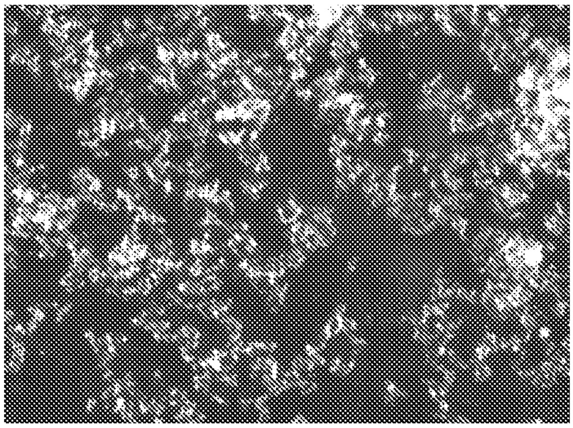

**B**

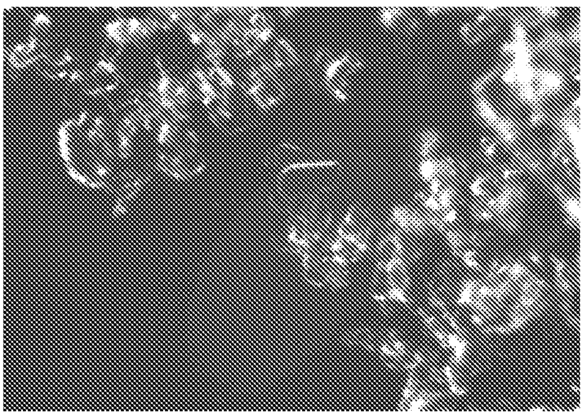

**C**

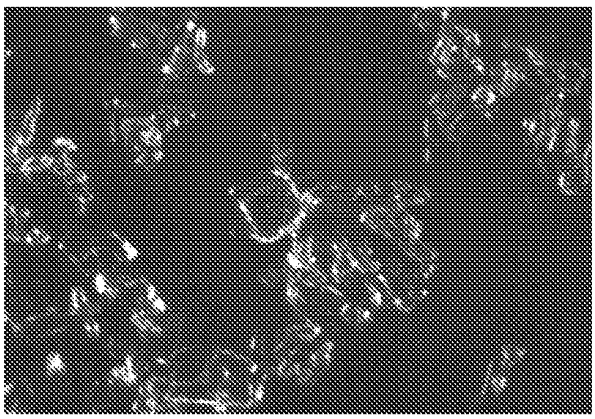

**D**

**Fig.2**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20080251169 A1
- WO 2010146170 A
- US 20080248578 A
- WO 04035018 A


### In der Beschreibung aufgeführte Nicht-Patentliteratur


- **S. BAJ et al.** *Green Chemistry,* 2006, vol. 8, 292-295
- Ionic Liquids in Synthesis. WILEY-VCH, 2003